# EUROPEAN PATENT SPECIFICATION

(11) **EP 1 146 033 B1**
(45) Date of publication and mention of the grant of the patent: **23.12.2009**
(21) Application number: 01106425.0
(22) Date of filing: 22.03.2001
(51) Int. Cl.: C07C 49/84, C11D 3/50, A61K 8/18

(54) **Fragrance precursors**
Riechstoffvorläufer
Précurseurs de parfum

(30) Priority: 10.04.2000 EP 00107680
(43) Date of publication of application: 17.10.2001
(73) Proprietor: Givaudan SA, 1214 Vernier-Genève (CH)
(72) Inventor: Gautschi, Markus, 4314 Zeiningen (CH); Plessis, Caroline, 72330 Yvre le Polin (FR); Derrer, Samuel, 8117 Fällanden (CH)
(74) Representative: Givaudan Patents

(56) References cited:
- EP-A- 0 983 990
- HONDA, YUTAKA ET AL: "A general synthetic method of chiral 2-arylalkanoic esters via thermal 1,2-rearrangement" BULL. CHEM. SOC. JPN. (1987), 60(3), 1027-36 , XP001042191
- UMEZAWA, TOSHIAKI ET AL: "Incorporation of 18O-water into the C.alpha. but not the C.beta. position in degradation of a.beta.-O-4 lignin substructure model by Phanerochaete chrysosporium" AGRIC. BIOL. CHEM. (1984), 48(7), 1917-21 , XP001042192
- ARIMOTO, HIROKAZU ET AL: "Synthesis and absolute stereochemistry of tanzawaic acid (GS-1302)" TETRAHEDRON LETT. (1998), 39(51), 9513-9516 , XP002186608
- ZHU, GUOXIN ET AL: "Practical Syntheses of.beta.-Amino Alcohols via Asymmetric Catalytic Hydrogenation" J. ORG. CHEM. (1998), 63(23), 8100-8101 , XP002186609
- MCGARVEY, GLENN J. ET AL: "A trialkylstannane -mediated approach to acyloin products" J. ORG. CHEM. (1985), 50(23), 4655-7 , XP002186610

## Description

The present invention relates to fragrance precursors for a fragrant ketone and a fragrant ester.

A principal strategy currently employed in imparting odors to consumer products is the admixing of the fragrance directly into the product. There are, however, several drawbacks to this strategy. The fragrance material can be too volatile and/or too soluble, resulting in fragrance loss during manufacturing, storage, and use. Many fragrance materials are also unstable over time. This again results in loss during storage.

In many consumer products it is desirable for the fragrance to be released slowly over time. Micro-encapsulation and inclusion complexes with cyclodextrins have been used to help decrease volatility, improve stability and provide slow-release properties. However, these methods are for a number of reasons often not successful. In addition, cyclodextrins can be too expensive.

It is therefore desirable to have a fragrance delivery system which is capable of releasing the fragrant compound or compounds in a controlled manner, maintaining a desired smell over a prolonged period of time.

Precursors for the delivery of organoleptic compounds, especially for flavors, fragrances and masking agents, is described in EP-A 0 936 211. This delivery system releases one or more odoriferous compounds upon exposure to light and/or UV irradiation. Using this system in various consumer products leads to a prolonged perception of the fragrant compound(s) to be released.

WO 99/60990 describes fragrance precursors which release fragrant alcohols, aldehydes or ketones upon exposure to light. Perfuming compositions comprising these fragrance precursors can be used in various consumer products such as detergents, fabric softeners, household products, hair-care products etc..

Many fragrant compounds with odors accepted by the public are esters of high volatility resulting in a short period of perceivable odor. Such esters are fast hydrolyzed in alkaline environment, thereby loosing the fragrant characteristic. Therefore, they are of limited use for laundry products.

Object of the present invention is to provide non volatile precursors for volatile fragrant esters.

A further object of the present invention is to provide fragrance precursors which are stable in alkaline environment, especially in laundry products.

Also an object of the present invention is to provide fragrance precursors with high substantivity.

A further object of the present invention is to provide fragrance precursors which are activated and cleaved by light. A further object of the present invention is to provide fragrance precursors which are activated and cleaved by light.

Also an object of the present invention is to provide fragrance precursors with slow release properties.

The present invention relates to the use as a fragrance precursor of a compound of formula I 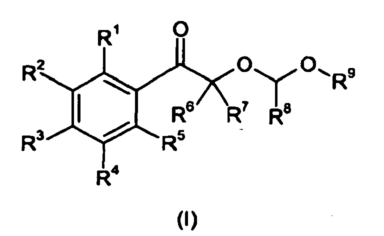 which upon exposure to light, and in particular daylight, release a fragrant ketone of formula II 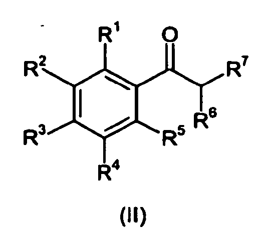 and a fragrant ester of formula III 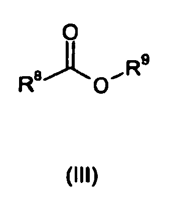 wherein
R¹ to R⁵ represent independently H, -NO₂, branched or linear C₁-C₆-alkyl, C₁-C₆-alkenyl, C₁-C₆-alkinyl or C₁-C₄-alkoxy, R¹ and R², R² and R³, R³ and R⁴ and R⁴ and R⁵ may form together one or two aliphatic or aromatic rings, these rings may optionally contain branched or linear C₁-C₄-alkyl, C₁-C₄-alkenyl or C₁-C₄-alkinyl residues, and the above rings and residues may comprise one or more oxygen atoms,
R⁶ and R⁷ are independently H, branched or linear C₁-C₆-alkyl, C₁-C₆-alkenyl, C₁-C₆-alkinyl, and R⁶ or R⁷ may form with either R¹ or R⁵ a carbocyclic ring optionally substituted by an aliphatic residue.
R⁸ and R⁹ are the residues of an acid R⁸-COOHhaving up to 4 carbon atoms, but not only one carbon atom and an aliphatic alcohol R⁹OH having 2 to 20 carbon atoms, forming the fragrant ester of formula III.
Branched carbon chains also comprise multiple branched chains.
The present invention also relates to a compound of formula I.

The fragrance precursors of formula I release upon exposure to light volatile fragrant esters of formula III and fragrant ketones of formula II. Since the precursors of the invention are stable in alkaline environment and show high substantivity, they are excellently adapted for detergent and laundry use.

The fragrance precursors of the present invention are slowly cleaved when exposed to light, in particular daylight. Upon absorption of energy from said light, the phenacyl acetals undergo a Norrish Type II photoreaction which leads to the release of a fragrant ketone of formula II and a fragrant ester of formula III.

The release of the above mentioned fragrant compounds occurs for example upon exposure to sunlight penetrating through ordinary windows and being not particularly rich in UV irradiation. It is needless to say that upon exposure to bright sunlight, in particular outdoors, the release of the fragrant compounds of formula II and III will occur faster and to a greater extent than upon exposure to room light inside a building. The cleavage of the precursors of the present invention can also be initiated by an appropriate lamp, for example a sun tanning lamp.

It is known that phenacyl glycosides undergo a Norrish Type II photoreaction leading to gluconolactones and the corresponding phenacyl compound (Crich et al., Tetrahedron, 1995, 51, 11945-11952). However, it has not been described or suggested to use such phenacyl acetals as fragrance precursors, which are capable of releasing a fragrant ketone and a fragrant ester over a prolonged period.

The photoreaction of the fragrance precursors of formula I involves in a first step the absorption of light by the keto-group followed by abstraction of the acetal-H atom and subsequent cleavage of the resulting 1,4-diradical (Scheme A). It has been found that the aromatic residue of the fragrance precursors plays an important role in this photoreaction as it influences the absorption maximum λₘₐₓ of the keto-group. Therefore, the cleavage properties of the fragrance precursors can be modified by variation of the substituents R¹ to R⁵.

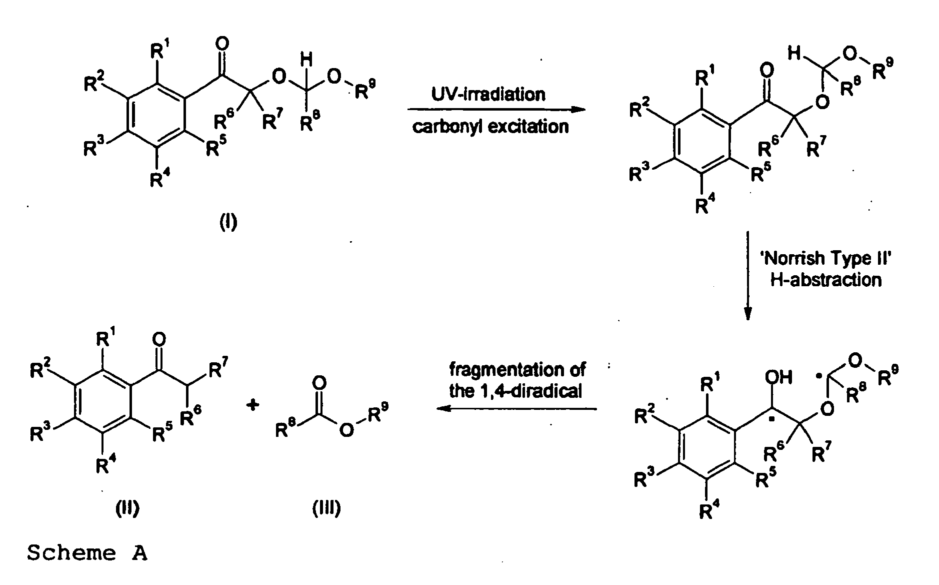

Fragrant aryl alkyl ketones of formula II are well known to those skilled in the art. A fragrant ketone of formula II is a compound known to a person skilled in the art as being a useful ingredient for the formulation of perfumes or perfumed articles. Non-limiting examples of said aryl alkyl ketones are acetanisole (1-(4-methoxyphenyl)-ethanone) [Givaudan Roure (International) SA, Vernier, Switzerland], acetophenone (1-phenyl-ethanone) [Haarmann & Reimer GmbH, Germany], Crysolide^{®} (4-acetyl-6-tert-butyl-1,1-dimethyl-indan) [Givaudan Roure (International) SA, Vernier, Switzerland], dimethyl acetophenone (1-(2,4-dimethylphenyl)-ethanone) [Fluka AG, Buchs, Switzerland], Fixolide^{®} (1-(5,6,7,8-tetrahydro-3,5,5,6,8,8-hexamethyl-2-naphthalenyl-ethanone) [Givaudan Roure (International) SA, Vernier, Switzerland], Florantone T^{®} (1-(5,6,7,8-tetrahydro-2-naphthalenyl)-ethanone) [Takasago Perfumery Co., Japan], Grassenone 34^{®} (3-methyl-1-(4-methylphenyl)-4-hexen-1-one) [Keemia Institute, Tallin USSR], isopropylindanone (2-(1-methylethyl)-indanone) [Givaudan Roure (International) SA, Vernier, Switzerland], Lavonax^{®} (1-phenyl-4-penten-1-one) [International Flavors & Fragrances, USA], Musk F (5-acetyl-1,1,2,3,3-pentamethyl-indane) [CNNP], Musk ketone^{®} (4-tert-butyl-3,5-dinitro-2,6-dimethyl-acetophenone) [Givaudan Roure (International) SA, Vernier, Switzerland], Novalide^{®} (1,6,7,8-tetrahydro-1,4,6,6,8,8-hexamethyl-indacen-3(2H)-one) [Givaudan Roure (International) SA, Vernier, Switzerland], Oranger Crystals^{®} (1-(2-naphthalenyl)-ethanone) [Givaudan Roure (International) SA, Vernier, Switzerland], Orinox^{®} (1-[4-(1,1-dimethylethyl)-2,6-dimethylphenyl]-ethanone) [Polak's Frutal Works BV, Netherlands], Phantolide^{®} (1-(2,3-dihydro-1,1,2,3,3,6-hexamethyl-1H-inden-5-yl-ethanone) [Polak's Frutal Works BV, Netherlands], propiophenone (1-phenyl-propanone) [Haarmann & Reimer GmbH, Germany], Traseolide 100^{®} (1-[2,3-dihydro-1,1,2,6-tetramethyl-3-(1-methylethyl-1H-inden-5-yl-ethanone) [Quest International, Netherlands], Vernolide^{®} (1-(5,6,7,8-tetrahydro-3',5',5',8',8'-pentamethyl-2-naphthalenyl)-ethanone) [Givaudan Roure (International) SA, Vernier, Switzerland], Versalide^{®} (1-(5,6,7,8-tetrahydro-3'-ethyl,5',5',8',8'-tetramethyl-2-naphthalenyl)-ethanone) [Givaudan Roure (International) SA, Vernier, Switzerland], Vitalide^{®} (1-(hexahydrodimethyl-1H-benzindenyl)-ethanone) [Takasago Perfumery, Japan].

It is obvious to the person skilled in the art that the above list is illustrative and that the present invention relates to many other fragrant ketones of formula II. Additional fragrant ketones of formula II are e.g. described in "Perfume and Flavor Chemicals", S. Arctander Ed. , Vol. I & II, Allured Publishing Corporation, Carol Stream, USA, 1994 and in K. Bauer, D. Garbe and H. Surburg, Eds., Common Fragrance and Flavor Materials, Wiley-VCH, 3rd Edition, Weinheim, 1997.

Fragrance esters of formula III, represent an important class of perfumery raw materials and comprise compounds of a great structural variety. Fragrance esters of formula III contribute to the odor and aroma of nearly all fruits and are known to be useful ingredients for the formulation of perfumes or perfumed articles. In the following a non-limiting list of such esters are given as examples.

Most of the aliphatic esters of formula III are either acetates or comprise ethanol as the alcohol component. Examples for such esters of formula III include amyl butyrate, butyl 2-methylpentanoate, 3,7-dimethyloctan-3-yl acetate, ethyl 2-methylbutyrate, hexyl acetate, hexyl isobutyrate and isopropyl 2-methylbutyrate.

The lower fatty acid esters of acyclic terpene alcohols, e.g. geraniol, linalool and citronellol, and of cyclic terpene alcohols, e.g. menthol, α-terpineol, borneol and guaiyol, are important both as fragrance and as flavor substances and are envisaged as esters of formula III.

Various cycloaliphatic esters of formula III are widely used perfumery chemicals, non-limiting examples are Agrumex^{®} (2-tert-butylcyclohexyl acetate) [Haarmann & Reimer GmbH, Germany], Vertenex^{®} (4-tert-butylcyclohexyl acetate) [International Flavors & Fragrances, USA], Verdylacetate^{®} (4,7-Methano-3a,4,5,6,7,7a-hexahydro-5(6)-indenyl acetate) [Givaudan Roure (International) SA, Vernier, Switzerland], Givescone^{®} (ethyl 2-ethyl-6,6-dimethyl-2-cyclohexenecarboxylate and ethyl 2,3,6,6-tetramethyl-2-cyclohexenecarboxylate) [Givaudan Roure (International) SA, Vernier, Switzerland], Cyclogalbanat^{®} (allyl cyclohexyloxyacetate) [DRAGOCO Gerberding & Co. AG, Germany], Methyl jasmonate^{®} (3-oxo-2-(cis-pentenyl)cyclopentaneacetic acid methyl ester) [Firmenich S.A., Switzerland] and Hedion^{®} (methyl (3-oxo-2-pentylcyclopentyl)acetate) [Firmenich S.A., Switzerland].

Other important esters of formula III used in perfumery are those derived from araliphatic alcohols and aliphatic acids. They have characteristic odor properties. Important esters that fall into this category are e.g. benzyl acetate, phenethyl acetate, α,α-dimethylphenethyl acetate and cinnamyl acetate.

Many of the esters of formula III described above, which are of pleasant odor, have a rather high volatility. This is especially true for aliphatic esters exhibiting typical fruity odors and for lower fatty acid esters of acyclic terpene alcohols having pleasant citrusy, floral odors. An example of such a volatile ester is e.g. cis-3-hexenyl acetate. Cis-3-hexenyl acetate applied to a surface of, for example, a fabric using a fabric softener in the rinsing cycle of the washing process, can only be perceived over a short period of time of one or two hours, depending on the concentration of cis-3-hexenyl acetate in the fabric softener.

The fragrance precursors of the present invention are not, or only slightly, volatile. The fragrant ketone of formula II and the fragrant ester of formula III are released only upon exposure to light, and especially daylight. The photochemical cleavage provides over days and weeks perceptible amounts of the fragrant compounds. The period depends interalia on the amount or concentration of the precursor applied, the duration of exposure to light, its intensity and its wavelength.

Fragrance esters of formula III are prone to undergo hydrolysis into an acid of formula R⁸COOH and an alcohol of formula R⁹OH, especially in alkaline products. Therefore many fragrance accords comprising such esters, e.g. fruity accords, cannot be imparted to such products.

Today's consumers select a certain product not only based on performance but also based on the odor. From the foregoing it is evident that products for introducing a variety of fragrance accords to products having alkaline pH are desirable. The fragrance precursors of the present invention have the advantage that they are not or only slightly volatile and chemically stable in consumer products having alkaline and neutral pH. A precursor of formula I added to a powder detergent, is stable in the detergent powder throughout storage. During the washing cycle (alkaline pH) and the rinsing cycle (neutral pH) the precursor is deposited on the fabric surface. It is only upon exposure of the fabric to light, for example during line drying in the sun, that the release of the fragrant ketone of formula II and the fragrant ester of formula III is started.

It has been mentioned above that esters of formula III, and especially the aliphatic ones, are rather volatile compounds. Furthermore, they are water soluble and are, therefore, lost to some extent during the washing/rinsing cycle if introduced directly into detergents.

The fragrance precursors of formula I have the advantage that they have good substantivity on different substrates, especially on fabrics. Furthermore, the precursors are not or only slightly volatile, thus no loss occurs during storage. With the precursors of the present invention highly volatile esters of formula III with low substantivity are successfully applied to achieve a long lasting pleasant odor. The volatile esters are produced in situ after application of the precursors of formula I onto a fabric during the washing cycle.

In the precursors of the invention the moiety derived from a fragrant ketone of formula II brings three advantages: it introduces stability as well as substantivity to the precursors of formula I and upon activation by light exhibits fragrant properties.

The fragrance precursors of the present invention are advantageously prepared via two methods. Both methods use an α-hydroxy-ketone as starting material. The latter is prepared by bromination of the corresponding fragrant ketone followed by sodium formate treatment and subsequent hydrolysis shown in scheme I:

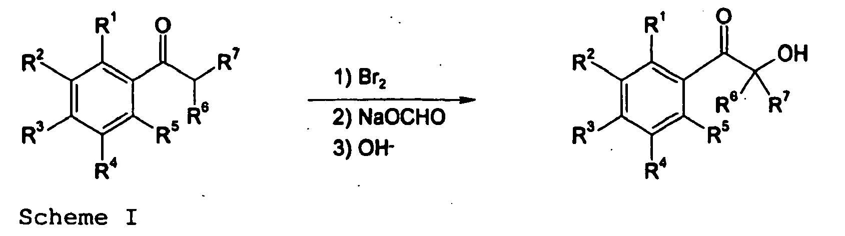

Then according to the first method the α-hydroxy-ketone intermediate is reacted under acid conditions with a vinyl ether to the desired precursor of formula I. The vinyl ether is obtained via the acetal of an aldehyde R⁸CHO and an alcohol R⁹OH. The synthesis is illustrated in scheme II:

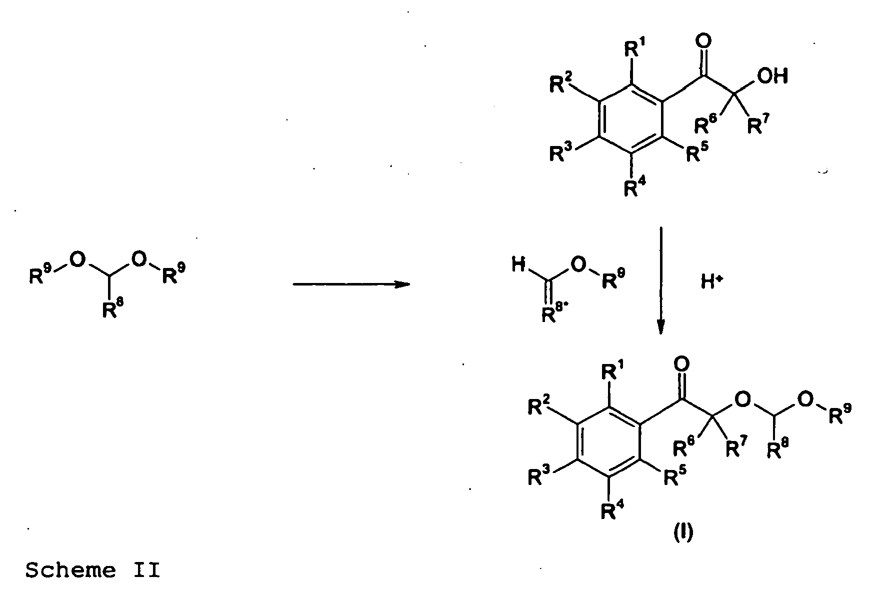

According to the second method the α-hydroxy-ketone is transformed to the corresponding vinyl ether using a Hg catalyst. The vinyl ether is then coupled with the alcohol R⁹OH from which the fragrant ester of formula III is derived. This method allows for the use of a great variety of alcohols, i.e. residues R⁹ especially for allylic residues. The synthesis via this route is illustrated in scheme III:

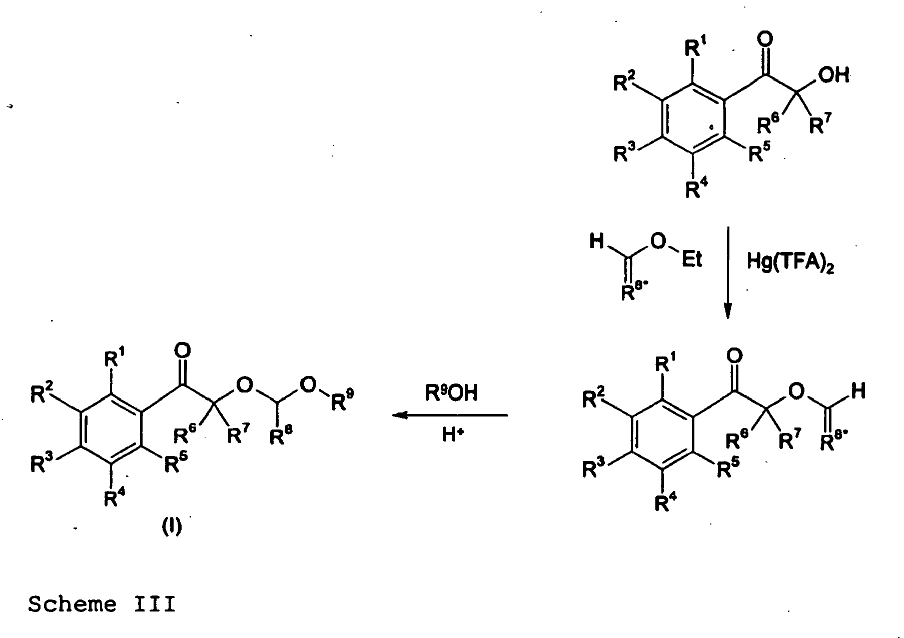

Preferred precursors of the present invention are compounds releasing an aliphatic ester of formula III wherein R⁸ is the residue of an aliphatic acid having 1 to 4 carbon atoms and R⁹ is the residue of an aliphatic alcohol having 2 to 20 carbon atoms. Most preferred precursors are those releasing an ester derived from acetic acid, i.e. wherein R⁸ is -CH₃.

Other preferred precursors include compounds wherein R⁸ is the residue of an aliphatic acid having 5 to 20 carbon atoms and R⁹ is the residue of an aliphatic alcohol having 1 to 5 carbon atoms. Most preferred compounds are those releasing an ester derived from ethanol, i.e. wherein R⁹ is -CH₂CH₃.

Other preferred precursors include compounds wherein R⁸ is the residue of an aliphatic acid having 1 to 4 carbon atoms and R⁹ is the residue of a terpene alcohol having 10 to 20 carbon atoms. Most preferred compounds are those wherein the alcohol is a monoterpene alcohol.

Other preferred precursors include compounds wherein R⁸ is the residue of a cycloaliphatic acid having 5 to 20 carbon atoms and R⁹ is the residue of an aliphatic alcohol having 1 to 5 carbon atoms. Most preferred compounds are those wherein the alcohol is ethanol.

Other preferred precursors include compounds wherein R⁸ is the residue of an aliphatic acid having 1 to 4 carbon atoms and R⁹ is the residue of an araliphatic alcohol having more than 5 carbon atoms. Most preferred precursors are those releasing an ester derived from acetic acid, wherein R⁸ is -CH₃.

Other preferred precursors include compounds wherein at least one of the residues R⁶ or R⁷ = H. Most preferred are compounds wherein R⁶ and R⁷ = H. Upon cleavage of these precursors a fragrant ketone of formula II is released wherein said ketone is an aryl methyl ketone.

Other preferred precursors include compounds wherein R⁶ and R⁷=H and R¹ to R⁵ represent independently hydrogen, -NO₂, linear or branched C₁-C₆ alkyl, alkenyl, alkinyl, and C₁-C₄ alkoxy. Most preferred compounds are those releasing a fragrant ketone of formula II wherein the fragrant ketone is selected from 1-phenyl-ethanone, 2,4-dimethylphenylethanone, 1-[4-(1,1-dimethylethyl)-2,6-dimethylphenyl]-ethanone, 1-(4-tert-butyl-3,5-dinitro-2,6-dimethyl)-ethanone and 1-(4-methoxyphenyl)-ethanone. Other preferred precursor include compounds wherein R¹ and R², R² and R³, R³ and R⁴, R⁴ and R⁵ form together an aliphatic or aromatic ring, wherein this ring may optionally contain substituted or unsubstituted C₁-C₄ alkyl, alkenyl, alkinyl residues and may comprise one or more oxygen atoms. Most preferred compounds are those releasing a fragrant ketone of formula II wherein the fragrant ketone is selected from 1-(2-naphtalenyl)-ethanone, 4-acetyl-6-tert-butyl-1,1-dimethyl-indan, 1-(5,6,7,8-tetrahydro-3,5,5,6,8,8-hexamethyl-2-naphthalenylethanone, 1-(5,6,7,8-tetrahydro-3',5',5',8',8'-pentamethyl-2-naphthalenyl)-ethanone, 1-(5,6,7,8-tetrahydro-3'-ethyl-5',5',8',8'-tetramethyl-2-naphthalenyl)-ethanone,1-(2,3-dihydro-1,1,2,3,3,6-hexamethyl-1H-inden-5-yl-ethanone, 1-[2,3-dihydro-1,1,2,6-tetramethyl-3-(1-methylethyl-1H-inden-5-yl-ethanone, 5-acetyl-1,1,2,3,3-pentamethyl-indane, 1-(5,6,7,8-tetrahydro-2-naphthalenyl)-ethanone.

Since the compounds of formula I, upon exposure to light are cleaved and provide a fragrant ketone of formula II and a fragrant ester of formula III, they permit the development of useful consumer products with enhanced fragrant properties, especially having long lasting pleasant odor. Therefore, the present invention also relates to the use of all compounds of formula I as precursors for fragrant compounds.

The fragrance precursors of the present invention can be used in any product in which a prolonged and defined release of the above mentioned fragrant compounds is desired. Therefore, these precursors are especially useful in functional perfumery, in products which are exposed to sunlight, during or after application.

The compounds of the present invention can act as fragrance precursors in functional and fine perfumery i.e. in fine fragrances, industrial, institutional, home and personal care products. Industrial, institutional and home cleaning products to which the fragrance precursors can be added are all kinds of detergents, window cleaners, hard surface cleaners, all purpose cleaners and furniture polishes. The products can be liquids or solids, such as powders or tablets. Fabrics and surfaces treated with a product comprising a fragrance precursor of the present invention will diffuse a fresh and clean odor upon exposure to light much longer than when cleaned with a conventional cleaner. Fabrics or cloths washed with such detergents will release the fragrant compounds even after having been stored for weeks in a dark place, e.g. a wardrobe.

The precursors of the present invention are also useful for application in all kinds of body care products. Especially interesting products are hair care products, for example shampoos, conditioners and hairsprays and skin care products such as cosmetic products and especially sun protection products.

The above mentioned examples are of course only illustrative and non-limiting. Many other products to which the precursors of the present invention may be added include soaps, bath and shower gels, deodorants and even perfumes and colognes.

The fragrance precursors of the present invention can be used alone or in combination with other fragrance ingredients, solvents or adjuvants known to those skilled in the art. Such ingredients are described, for example, in "Perfume and Flavor Chemicals", S. Arctander, Ed., Vol. I & II, Allured Publishing Corporation, Carol Stream, USA, 1994 and include fragrance compounds of natural or synthetic origin and essential oils of natural products.

The amounts in which the precursors of formula I are incorporated in the various above-mentioned products vary within a wide range. The amounts depend on the nature of the fragrant compounds to be released, the nature of the product to which the precursors are added and the desired olfactory effect. The amounts used also depend on the co-ingredients in a given composition when the precursors of the present invention are used in admixture with perfuming co-ingredients, solvents or adjuvants. Typical concentrations are in the order of 0.01% to 5% by weight of the products.

The following non-limiting examples further illustrate the embodiments of the invention.

The following chemicals were obtained from commercial sources: bromo-acetonaphtone, bromo-acetanisole, sodium formate, trifluoroacetic acid, ethyl vinyl ether, mercury trifluoroacetate, 2-phenyl-ethanol, cis-3-hexenol, 3,5,5-trimethyl-hexanol, hexanol, 3-phenyl-propanol citronellol, 3,7-dimethyl-3-octanol, 4-tert-butyl-cyclohexanol, β-methoxy-styrene.
α-Bromo-Fixolide was prepared from Fixolide^{®} according to R.M. Cowper, L.H. Davidson, Org. Synth. Coll. Vol. II, 1943, 480-481.
NMR: values of coupling constants *J* are given in Hertz (Hz).

### Example 1

### Preparation of Phenacyl acetals

### 1. General procedure for the preparation of hydroxy-acetophenones

A suspension of the corresponding bromo-acetophenone (0.05 mmol) and sodium formate (17 g, 0.25 mol, 5 eq.) in aqueous ethanol (85%, 150 ml) was heated at reflux until completion of the reaction (TLC). Most of the ethanol was evaporated and the mixture partitioned between MTBE (80 ml) and water (70 ml). The organic phase was separated and washed with aq. NaHCO₃ (sat.) and brine. Removal of the solvent in vacuo, after drying over MgSO₄, afforded a crude product as a solid which was recrystallised from ethanol.

### 2-Hydroxy-1-(4-methoxy-phenyl)-ethanone

Obtained according to the general procedure.
**mp** 104-105 °C.
**¹H-NMR** (400 MHz, CDCl₃): 3.48 (t, 1H, *J* 4); 4.82 (d, 2H, *J* 4); 6.95-7.0 (m, 2H); 7.85-7.95 (m, 2H).
**IR** (*v*ₘₐₓ, cm⁻¹, neat): 3415m, 2929w, 1672s, 1603s.
**MS [*m*/*z* (EI)]**: 166 (M⁺, 4), 155 (100), 77 (28).

### 1-(3,5,5,6,8,8-Hexamethyl-5,6,7,8-tetrahydro-naphthalen-2-yl)-2-hydroxy-ethanone

Obtained according to the general procedure.
**mp** 81-82 °C.
**¹H-NMR** (400 MHz, CDCl₃): 1. 0 (d, 3H, *J* 6.8) ; 1.08 (s, 3H); 1.26 (s, 3H); 1.31 (s, 3H); 1.33 (s, 3H); 1.41 (dd, 1H, *J* 13.2, 2.4); 1.63 (dd, 1H, *J* 13.2, 13.2); 1.8-1.95 (m, 1H); 2.54 (s, 3H); 4.76 (s, 2H); 7.26 (s, 1H); 7.57 (s, 1H).
**IR** (*v*ₘₐₓ, cm⁻¹, neat): 3447w, 2963m, 2911m, 1675s, 1607w.
**MS [*m*/*z* (EI)] :** 274 (M⁺, 3), 243 (100).

### 2-Hydroxy-1-naphthalen-2-yl-ethanone

Obtained according to the general procedure.
**mp** 114-115 °C.
**¹H-NMR** (400 MHz, CDCl₃): 3.59 (t, 1H, *J* 4.4); 5.02 (d,2H, *J* 4.4); 7.55-7.7 (m, 2H); 7.85-8.0 (m, 4H); 8.43 (s, 1H). **IR** (*v*ₘₐₓ, cm⁻¹, neat): 3428m, 3391m, 3051w, 2931w, 1680s, 1627m.
**MS [m/z (EI)]:** 186 (M⁺, 12), 155 (75), 127 (100), 40 (26), 28 (41).

### 2. General procedure for the preparation of alkyl vinyl ethers

A solution of the alcohol (0.1 mol) and mercury(II) trifuoroacetate (4 mmol, 0.04 eq.) in ethyl vinyl ether (50 ml, 1 mol, 5 eq.) was heated at reflux until completion of the reaction (TLC, GC). The ethyl vinyl ether was evaporated and the residue diluted with MTBE and poured into aq. NaHCO₃ (sat.). The separated aqueous phase was extracted with MTBE and the combined organic layers were washed with brine and dried over MgSO₄. After concentration, the crude oil was distilled under reduced pressure to afford the desired product as a colorless oil.

### Hexyloxy-ethene

Obtained according to the general procedure.
**bp_{170mbar}** 89°C.
**¹H-NMR** (400 MHz, CDCl₃): 0.9 (t, 3H, *J* 6.8);1.25-1.42 (m, 6H); 1.6-1.7 (m, 2H); 3.67 (t, 2H, *J* 6.8); 3.96 (dd, 1H, *J* 6.8, 2); 4.16 (dd, 1H, *J* 14.4, 2); 6.46 (dd, 1H, *J* 14.4, 6.8).
**IR** (*v*ₘₐₓ, cm⁻¹, neat): 3119w, 2957s, 2932s, 2861m, 1740w, 1636m, 1611s.
**MS [*m*/*z*** (EI)]: 128 (M⁺, 1), 56 (34), 55 (23), 43 (100), 41 (39).

### (2-Vinyloxy-ethyl)-benzene

Obtained according to the general procedure.
**¹H-NMR** (400 MHz, CDCl₃) : 2.96 (t, 2H, *J* 7.2); 3.88 (t, 2H, *J* 7.2); 3.99 (dd, 1H, *J* 6.8, 2); 4.18 (dd, 1H, *J* 14.4, 2); 6.46 (dd, 1H, *J* 14.4, 6.8); 7.19-7.32 (m, 5H).
**IR** (*v*ₘₐₓ, cm⁻¹, neat): 3028m, 2947m, 2872m, 1636m, 1615s.
**MS [m/z (EI)]**: 148 (M⁺, 1), 105 (100), 104 (36), 79 (21), 77 (21).

### (3,5,5-Trimethyl-hexyloxy)-ethene

Obtained according to the general procedure.
**bp_{45mbar}** 95 °C.
**¹H-NMR** (400 MHz, CDCl₃): 0.9 (s, 9H); 0.95 (d, 3H, *J* 6.4); 1.05-1.27 (m, 2H); 1.42-1.52 (m, 1H); 1.6-1.7 (m, 2H); 3.68 (t, 2H, *J* 6.4); 3.96 (dd, 1H, *J* 7, 2); 4.16 (dd, 1H, *J* 15, 2); 6.46 (dd, 1H, J 15, 7).
**IR** (*v*ₘₐₓ, cm⁻¹, neat): 2955s,2870m, 1649m, 1635m, 1610m.
**MS [*m*/*z* (EI)]:** 170 (M⁺, 1), 71 (23),70 (24), 69 (21), 57 (100), 41 (22).

### 1-Vinyloxy-hex-3(Z)-ene

Obtained according to the general procedure.
**bp_{140mbar}** 86 °C.
**¹H-NMR** (400 MHz, CDCl₃): 0.97 (t, 3H, *J* 7.2); 2.0-2.1 (m, 2H); 2.37-2.45 (m, 2H); 3.68 (t, 2H, *J* 7.2); 3.98 (dd, 1H, *J* 6.8, 2); 4.18 (dd, 1H, *J* 14.4, 2); 5.3-5.4 (m, 1H); 5.47-5.55 (m, 1H); 6.46 (dd, 1H, *J* 14.4, 6.8).
**IR** (*v*ₘₐₓ, cm⁻¹, neat): 3011w, 2965m, 2934m, 2874m, 1740w, 1636m, 1613m.
**MS [*m*/*z* (EI)]:** 126 (M⁺, 1), 83 (21), 70 (45), 67 (34), 55 (100), 41 (45).

### (1-Ethyl-1,5-dimethyl-hexyloxy) -ethene

Obtained according to the general procedure.
**bp_{15mbar}** 88-90°C.
**¹H-NMR** (400 MHz, CDCl₃): 0.85-0.9 (m, 9H); 1.12-1.6 (m, 9H); 1.18 (s, 3H); 4.01 (d, 1H, *J* 6.4); 4.40 (dd, 1H, *J* 13.6, 0.4); 6.41 (dd, 1H, *J* 13.6, 6.4).
**IR** (*v*ₘₐₓ, cm⁻¹, neat): 3010w, 2940s, 2860m, 1625s.
**MS [*m*/*z* (EI)]:** 184 (M⁺, 1), 85 (51), 71 (59), 69 (20), 57 (100), 55 (31), 43 (83), 41 (32), 29 (23).

### 2,6-Dimethyl-8-vinyloxy-oct-2-ene

Obtained according to the general procedure.
**bp_{15mbar}** 98 °C.
**¹H-NMR** (400 MHz, CDCl₃): 0.82 (d, 3H, *J* 8); 1.05-1.7 (m, 5H); 1.51 (s, 3H); 1.59 (s, 3H); 1.8-2.0 (m, 2H); 3.57-3.65 (s, 2H); 3.87 (dd, 1H, *J* 8, 4); 4.07 (dd, 1H, *J* 16, 4); 4.97-5.05 (m, 1H); 6.37 (dd, 1H, *J* 16, 8).
**IR** (*v*ₘₐₓ, cm⁻¹, neat): 2960m, 2927w, 1636w, 1610m.
**MS [*m*/*z* (EI)]:** 182 (M⁺, 1), 181 (1), 123 (22), 95 (36), 82(28), 81(37), 69 (100), 68 (22), 67 (33), 55 (47), 41 (64).

### (3-Vinyloxy-propyl)-benzene

Obtained according to the general procedure, after chromatography (SiO₂, EtOAc/Hexane) of the crude.
**¹H-NMR** (400 MHz, CDCl₃): 1.9-2.05 (m, 2H); 2.72 (t, 2H, *J* 7.6); 3.68 (t, 2H, *J* 6.4); 3.98 (dd, 1H, *J* 6.8, 2); 4.16 (dd, 1H, *J* 14.4, 2); 6.48 (dd, 1H, *J* 14.4, 6.8); 7.15-7.35 (m, 5H).
**IR** (*v*ₘₐₓ, cm⁻¹, neat): 3027w, 2946w, 2870w, 1636m, 1613s.
**MS [*m*/*z*** (EI)]: 162 (M⁺, 1), 118 (52), 117 (30), 91 (100).

### 1-t-Butyl-4-vinyloxy-cyclohexane

Obtained according to the general procedure.
**bp_{15mbar}** 95 °C.
**¹H-NMR** (400 MHz, CDCl₃): 0.8-0.9 (m, 9H); 0.95-1.1 (m, 2H); 1.1-1.45 (m, 4H); 1.5-1.6 (m, 1H); 1.75-1.85 (m, 1H); 1.9-2.13 (m, 2H); 3.57-3.67 (m, 0.6H); 3.95-4.05 (m, 1.4H); 4.28 (dd, 1H, *J* 14, 1.2);6.27-6.37 (m, 1H).
**IR** (*v*ₘₐₓ, cm⁻¹, neat): 2943s, 2865m, 1633m, 1607w.
**MS [*m*/*z* (EI)]:** 182 (M⁺, 4), 83 (46), 69 (23), 57 (100), 55 (23), 41 (25).

### 3. General procedure for the preparation of phenacyl acetals (I, fragrance precursors)

To a suspension of the α-hydroxy-acetophenone (20 mmol) in toluene (10 ml) was added the alkyl vinyl ether (2 eq.), followed by trifluoroacetic acid (2 or 3 drops, - 0.01 eq.). The mixture was heated at 50°C. When the reaction was finished (TLC), it was diluted with MTBE and poured into aq. NaHCO₃ (sat.). The aqueous phase was separated and extracted with MTBE, and the combined organic layers were washed with brine and dried over MgSO₄. The crude, obtained after evaporation of the solvents, was purified by chromatography (SiO₂, EtOAc/Hexane) to afford the desired product as a colorless to pale yellow oil.

### 2-(1-Ethoxy-ethoxy)-1-(4-methoxy-phenyl)-ethanone (1)

Obtained according to the general procedure without the use of solvent. No purification was required.
**¹H-NMR** (400 MHz, CDCl₃) : 1.19 (t, 3H, *J* 7.2); 1.4 (d, 3H, *J* 5.2); 3.5-3.7 (m, 2H); 3.87 (s, 3H); 4.77 (m, 2H); 4.91 (q, 1H, *J* 5.6); 6.9-7.0 (m, 2H); 7.9-8.0 (m, 2H).
**IR** (*v*ₘₐₓ, cm⁻¹, neat): 2977w, 1693m, 1601s, 1576m, 1512.
**UV** [λ(ε), nm, CH₂Cl₂]: 219 (11796), 273 (17127).
**MS [*m*/*z* (EI)]:** 237 (M⁺), 135 (100), 77 (26).

### 1-(4-Methoxy-phenyl)-2-(1-phenethyloxy-ethoxy)-ethanone (2)

Obtained according to the general procedure without the use of solvent.
**¹H-NMR** (200 MHz, CDCl₃) : 1.37 (d, 3H, *J* 5); 2.8-2.9 (m, 2H); 3.65-3.9 (m, 2H); 3.87 (s, 3H); 4.42-4.62 (m, 2H); 4.89 (q, 1H, *J* 5); 6.87-6.95 (m, 2H); 7.1-7.3 (m, 5H); 7.75-7.85 (m, 2H).
**IR** (*v*ₘₐₓ, cm⁻¹, neat): 2987m, 2936m, 2840m, 1693s, 1601s, 1575m, 1512m.
**UV** [λ(ε), nm, CH₂Cl₂]: 276 (15042).
**MS [m/z (EI)]:** 314 (M⁺), 150 (44), 135 (86), 105 (100), 77 (29).

### 2-(1-Hex-3(Z)-enyloxy-ethoxy)-1-(4-methoxy-phenyl)-ethanone (3)

Obtained according to the general procedure without the use of solvent.
**¹H-NMR** (200 MHz, CDCl₃): 0.95 (t, 3H, *J* 7.5); 1.4 (d, 3H, *J* 6); 1.95-2.15 (m, 2H); 2.25-2.4 (m, 2H); 3.4- 3.7 (m, 2H); 3.87 (s, 3H); 4.8 (m, 2H); 4.92 (q, 1H, *J* 6); 5.25-5.55 (m, 2H); 6.9-7.0 (m, 2H); 7.9-8.0 (m, 2H).
**IR** (*v*ₘₐₓ, cm⁻¹, neat): 2963m, 2934m, 2874m, 1695m, 1602s, 1576m, 1512m.
**UV** [λ(ε), nm, CH₂Cl₂]: 219 (11211), 273 (16231).
**MS [*m*/*z* (EI)]:** 292 (M⁺, 1), 150 (27), 135 (100), 83 (75), 55 (57).

### 1-(4-Methoxy-phenyl)-2-[1-(3,5,5-trimethyl-hexyloxy)-ethoxy]-ethanone (4)

Obtained according to the general procedure without the use of solvent.
**¹H-NMR** (200 MHz, CDCl₃): 0.8-0.95 (m, 3H); 0.86 (s, 9H); 1.0-1.35 (m, 3H); 1.41 (d, 3H, *J* 5); 1.45-1.7 (m, 2H); 3.4-3.7 (m, 2H); 3.89 (s, 3H); 4.65-4.7 (m, 2H); 4.9 (q, 1H, *J* 5); 5.05-5.1 (m, 1H); 6.9-7.0 (m, 2H); 7.9-8.0 (m, 2H).
**IR** (*v*ₘₐₓ, cm⁻¹, neat): 2954s, 1695m, 1602s, 1576m, 1512m.
**UV** [λ(ε), nm, CH₂Cl₂] : 219 (10941), 273 (15481).
**MS [m/*z* (EI)]:** 336 (M⁺), 135 (73), 71 (24), 70 (22), 69 (21), 57 (100), 41 (22).

### 2-(1-Hexyloxy-ethoxy)-1-(4-methoxy-phenyl)-ethanone (5)

Obtained according to the general procedure, but using Montmorillonite^{®} in refluxing toluene instead of TFA.
**¹H-NMR** (200 MHz, CDCl₃): 0.8-1.0 (m, 3H); 1.1-1.7 (m, 11H); 3.4-3.7 (m, 2H); 3.89 (s, 3H); 4.7-4.8 (m, 2H); 4.91 (q, 1H, *J* 6.2); 6.9-7.0 (m, 2H); 7.9-8.0 (m, 2H).

**IR** (*v*ₘₐₓ, cm⁻¹, neat): 2932s, 2859m, 1694m, 1601s, 1576m, 1512s.
**UV** [λ(ε), nm, CH₂Cl₂]: 219 (10656), 276 (15203).
**MS [*m*/*z* (EI)]:** 294 (M⁺), 135 (93), 85 (21), 56 (35), 55 (24), 43 (100), 41 (36).

### 1-(4-Methoxy-phenyl)-2-[1-(3-phenyl-propoxy)-ethoxy]-ethanone (6)

Obtained according to the general procedure without the use of solvent.
**¹H-NMR** (400 MHz, CDCl₃): 1.4 (d, 3H, *J* 5.2); 1.85-1.9 (m, 2H); 2.65-2.7 (m, 2H); 3.45-3.65 (m, 2H); 3.86 (s, 3H); 4.76 (m, 2H); 4.9 (q, 1H, *J* 5.2); 6.9-7.0 (m, 2H); 7.1-7.3 (m, 5H); 7.9-8.0 (m, 2H).
**IR** (*v*ₘₐₓ, cm⁻¹, neat): 2936w, 1693m, 1600s, 1575m, 1511m.
**UV** [λ(ε), nm, CH₂Cl₂]: 217 (18180), 273 (18826).
**MS [*m*/*z* (EI)]**: 328 (M⁺), 135 (51), 118 (45), 117 (29), 92 (20), 91 (100), 77 (22).

### 2-[1-(3,7-Dimethyl-oct-6-enyloxy)-ethoxy]-1-(4-methoxy-phenyl)-ethanone (7)

Obtained according to the general procedure.
**¹H-NMR** (400 MHz, CDCl₃): 0.8-0.95 (m, 3H); 1.1-1.2 (m, 1H); 1.25-1.45 (m, 5H); 1.5-1.7 (m, 8H); 1.9-2.05 (m, 2H); 3.45-3.7 (m, 2H); 3.87 (s, 3H); 4.7-4.82 (m, 2H); 4.9 (q, 1H, *J* 5.6); 5.05-5.1 (m, 1H); 6.9-7.0 (m, 2H); 7.9-8.0 (m, 2H).
**IR** (*v*ₘₐₓ, cm⁻¹, neat): 3534w, 2914m, 1694m, 1601s, 1576m, 1511m.
**UV** [λ(ε), nm, CH₂Cl₂]: 218 (13546), 273 (18063).
**MS [*m*/*z* (EI)]**: 348 (M⁺), 193 (42) , 135 (100) , 121 (31) , **83** (29), 81 (24), 69 (60), 41 (22).

### 1-(3,5,5,6,8,8-Hexamethyl-5,6,7,8-tetrahydro-naphthalen-2-yl)-2-(1-hexyloxy-ethoxy)-ethanone (8)

Obtained according to the general procedure without the use of solvent.
**¹H-NMR** (400 MHz, CDCl₃) : 0.87 (t, 3H, *J* 7.2); 0.99 (d, 3H, *J* 6.9); 1.06 (s, 3H); 1.15-1.45 (m, 20H); 1.5-1.7 (m, 2H); 1.8-1.95 (m, 1H); 2.48 (s, 3H); 3.4-3.65 (m, 2H); 4.68 (m, 2H); 4.89 (q, 1H, *J* 5.2); 7.21 (s, 1H); 7.55 (s, 1H).
**IR** (*v*ₘₐₓ, cm⁻¹,neat): 2960m, 2929m, 2871m, 1681m, 1607w, 1544w.
**UV** [λ(ε), nm, CH₂Cl₂]: 217 (20110), 257 (11478).
**MS [*m*/*z* (EI) ]:** 402 (M⁺), 243 (100), 85 (22), 43 (24).

### 1-(3,5,5,6,8,8-Hexamethyl-5,6,7,8-tetrahydro-naphthalen-2-yl)-2-(1-hex-3(Z)-enyloxy-ethoxy)-ethanone (9)

Obtained according to the general procedure without the use of solvent. No purification was required.
**¹H-NMR** (400 MHz, CDCl₃): 0.95 (t, 3H, *J* 6.8); 0.99 (d, 3H, *J* 6.8); 1.07 (s, 3H); 1.15-1.45 (m, 12H); 1.6-1.7 (m, 2H); 1.8-1.95 (m, 1H); 2.0-2.1 (m, 2H); 2.25-2.35 (m, 2H); 2.48 (s, 3H); 3.4-3.7 (m, 2H); 4.69 (m, 2H); 4.91 (q, 1H, *J* 5.2); 5.3-5.5 (m, 2H); 7.21 (s, 1H); 7.54 (s, 1H).
**IR** (*v*ₘₐₓ, cm⁻¹, neat): 2963s, 2931m, 1681m, 1608w, 1544w.
**UV** [λ(ε), nm, CH₂Cl₂]: 216 (21722), 258 (12495), 295 (2228).
**MS [*m*/*z* (EI)]:** 400 (M⁺, 1), 243 (100), 83 (28), 55 (24).

### 2-(1-Ethoxy-ethoxy)-1-(3,5,5,6,8,8-hexamethyl-5,6,7,8-tetrahydro-naphthalen-2-yl)-ethanone (10)

Obtained according to the general procedure without the use of solvent.
**¹H-NMR** (400 MHz, CDCl₃) : 0.94 (d, 3H, *J* 6.8); 1.0(s,3H); 1.15-1.45 (m, 15H); 1.55-1.7 (m, 2H); 1.8-1.95 (m, 1H); 2.47 (s, 3H); 3.5-3.75 (m, 2H); 4.68 (m, 2H); 4.89 (q, 1H, *J* 5.6); 7.21(s, 1H); 7.55 (s, 1H).
**IR** (*v*ₘₐₓ, cm⁻¹, neat): 2964s, 2929m, 1681m, 1607w,1544w.
**UV** [λ(ε), nm, CH₂Cl₂]: 217 (20799), 257 (11635).
**MS [*m*/*z* (EI)]:** 346 (M⁺), 243 (100).

### 2-(1-Hexyloxy-ethoxy)-1-naphthalen-2-yl-ethanone (11)

Obtained according to the general procedure.
**¹H-NMR** (400 MHz, CDCl₃): 0.86 (t, 3H); 1.2-1.4 (m, 6H); 1.43 (d, 3H, *J* 5.2); 1.5-1.6 (m, 2H); 3.45-3.7 (m, 2H); 4.9-5.02 (m, 3H); 7.52-7.65 (m, 2H); 7.85-8.05 (m, 4H); 8.47 (s, 1H).
**IR** (*v*ₘₐₓ, cm⁻¹, neat): 2930m, 2858w, 1697m, 1628w.
**UV** [λ(ε), nm, CH₂Cl₂]:250 (51217), 285 (9882).
**MS [*m*/*z* (EI)]:** 314 (M⁺), 155 (100), 127 (87), 56 (22), 43 (67), 41 (23).

### 2-[1-(3,7-Dimethyl-oct-6-enyloxy)-ethoxy]-1-naphthalen-2-yl-ethanone (12)

Obtained according to the general procedure.
**¹H-NMR** (400 MHz, CDCl₃): 0.8-0.95 (m, 3H); 1.1-1.2 (m, 1H); 1.25-1.5 (m, 2H); 1.43 (d, 3H, *J* 5.6); 1.5-1.7 (m, 8H); 1.57 (s, 3H); 1.66 (s, 3H); 1.85-2.05 (m, 2H); 3.45-3.75 (m, 2H); 4.9-5.0 (m, 3H); 5.02-5.1 (m, 1H); 7.52-7.65 (m, 2H); 7.85-8.05 (m, 4H); 8.47 (s, 1H).
**IR** (vₘₐₓ, cm⁻¹, neat): 2914m, 1698m, 1623w, 1597w.
**UV** [λ(ε), nm, CH₂Cl₂]: 250 (51252), 285 (9760).
**MS [*m*/*z* (EI)]:** 368 (M⁺), 213 (26), 155 (83), 142 (26), 127 (26), 83 (54), 81 (31), 69 (100), 57 (28), 55 (24), 41 (35).

### 2-[1-(1-Ethyl-1,5-dimethyl-hexyloxy)-ethoxy]-1-naphtalen-2-yl-ethanone (13)

Obtained according to the general procedure.
**¹H-NMR** (400 MHz, CDCl₃):0.8-0.9 (m, 9H); 1.1-1.65 (m, 15H); 4.9-5.02 (m, 2H); 5.27 (m, 1H); 7.55-7.65 (m, 2H); 7.85-8.05 (m, 4H); 8.49 (s, 1H).
**IR** (*v*ₘₐₓ, cm⁻¹, neat): 3520w, 2951m, 1699s, 1628m, 1597w.
**UV** [λ(ε), nm, CH₂Cl₂]: 250 (54132), 284 (10278).
**MS [*m*/*z* (EI)]:** 370 (M⁺, 2), 213 (32), 156 (21), 155 (100), 141 (55), 127 (65), 85 (53), 71 (60), 69 (23), 57 (74), 55 (32), 43 (74), 41 (34).

### 2-[1-(4-t-Butyl-cyclohexyloxy)-ethoxy]-1-naphtalen-2-yl-ethanone (14)

Obtained according to the general procedure. The two diastereoisomers could be separated by chromatography. *trans*-isomer :
**¹H-NMR** (400 MHz, CDCl₃): 0.82 (s, 9H); 0.9-1.05 (m, 3H); 1.15-1.35 (m, 2H); 1.42 (d, 3H, *J* 2.1); 1.7-1.8 (m, 2H); 1.95-2.1 (m, 2H); 3.75-3.6 (m, 1H); 4.95 (m, 2H); 5.1 (q, 1H); 7.5-7.65 (m, 2H); 7.85-8.05 (m, 4H); 8.5 (s, 1H). *cis*-isomer :
**¹H-NMR** (400 MHz, CDCl₃): 0.84 (s, 9H); 0.95-1.05 (m, 2H); 1.25-1.55 (m, 6H); 1.75-2.05 (m, 4H); 3.9-3.95 (m, 1H); 4.95 (m, 2H); 5.02 (q, 1H); 7.5-7.65 (m, 2H); 7.85-8.05 (m,4H); 8.5 (s, 1H).
**IR** (*v*ₘₐₓ, cm⁻¹, neat): 2939m, 2865m, 1698m, 1628w.
**UV** [λ(ε), nm, CH₂Cl₂]: 251 (43232), 287 (8289).
**MS [*m*/*z* (EI)]:** 368 (M⁺), 170 (28), 155 (39), 139 (38), 127 (31), 83 (53), 57 (100), 41 (27).

### 1-(4-Methoxy-phenyl)-2-(1-methoxy-2-phenyl-ethoxy)-ethanone (15)

Obtained according to the general procedure without the use of solvent.
**¹H-NMR** (400 MHz, CDCl₃): 3.0-3.05 (m, 2H); 3.35 (s, 3H); 3.88 (s, 3H); 4.76 (m, 2H); 4.8-4.85 (m, 1H); 6.9-6.95 (m, 2H); 7.15-7.25 (m, 5H); 7.9-7.95 (m, 2H).
**IR** (vₘₐₓ, cm⁻¹, neat): 2933 m, 2838m, 1692m, 1600s, 1575m, 1511s.
**UV** [λ(ε) , nm, CH₂Cl₂]: 218 (17292), 277 (13404)
**MS [*m*/*z* (EI)]:** 300 (M⁺), 209 (26), 149 (34), 135 (100), 134 (21), 121 (46), 91 (43), 77 (24).

### Example 2

### Photolysis of phenacyl acetals (I) in solutions

Photorelease assays were conducted on solutions (typical concentrations of precursors (I): 0.05% to 0.1% g/v) in organic solvents (preferably ethanol) or on cotton towels after deposition of the phenacyl acetals (I), as described below in the example 3.
The solutions were irradiated with a mercury lamp (150 W) in a borosilicate glass apparatus (Pyrex^{®} ) so as to limit the irradiation window to mainly the UVA and UVB spectrum of sun light. The alcoholic solution was irradiated for one hour and samples taken every 15 min to analyze the extent of the photolysis.

### Analysis

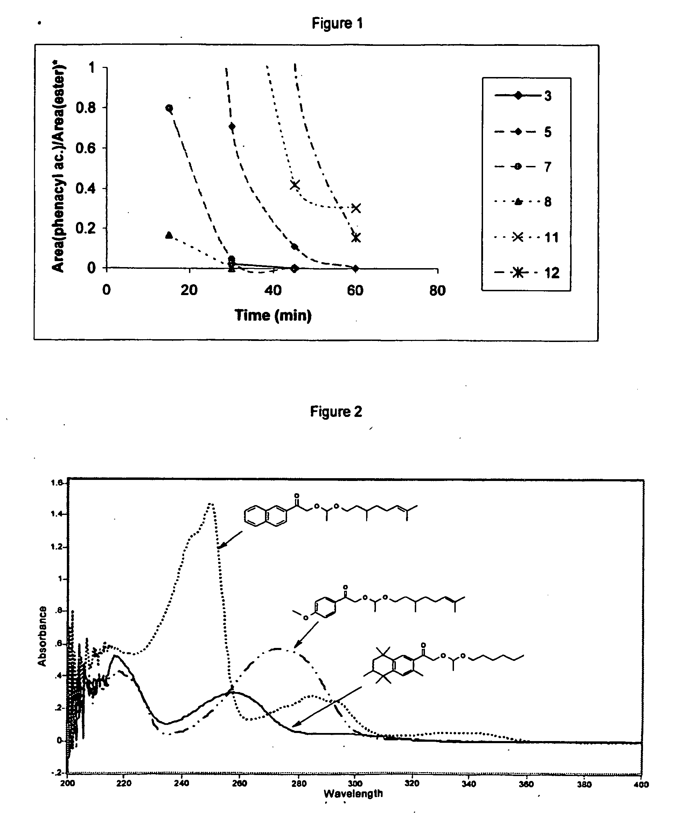

The presence of the aryl ketone (II) and ester (III) after photolysis in solutions was determined by using GC retention times. Samples (0.2 µl) were injected (on column injection) without further dilution. Gas chromatography-flame ionisation detection (GC-FID) was carried out with a Fisons-GC 8000series apparatus, using a *J*&*W Scientific* DB-5 capillary column (30m, 0.32mm id, 0.25µm film, He carrier gas, 85 kPa). The results are summarized in table 1.
Whereas precursors derived from Oranger Crystals® cleaved fairly slowly (Figure 1), those derived from acetanisole cleaved fast and Fixolide^{®} precursors even faster. The estimated half lives under the said conditions were inferred from the curves given in Figure 1. * The rates are calculated from the GC analysis (corresponding peak area).Representative UV spectra are shown in Figure 2.
t_{1/2} (Fixolide^{®}) = 15 min
t_{1/2} (Acetanisole) = 20-30 min
t_{1/2} (Oranger Crystals^{®})= 50-60 min

**Table 1: Release of aryl ketones (II) and esters (III) from phenacyl acetals (I) in solution upon irradiation with a mercury lamp**

| | STRUCTURE (I) | Fragrance Target | | UV-test* |
|---|---|---|---|---|
| | | aryl ketone (II) | ester (III) | |
| **1** | 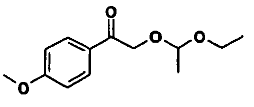 | acetanisole | (ethyl acetate) | ++ |
| **2** | 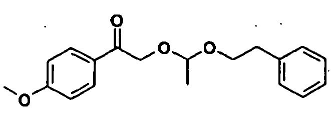 | acetanisole | phenethyl acetate | ++ |
| **3** | 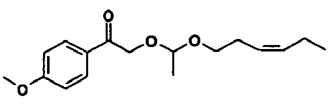 | acetanisole | *cis*-3-hexenyl acetate | ++ |
| **4** | 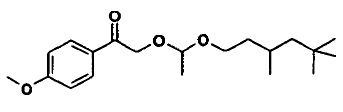 | acetanisole | nonanyl acetate | ++ |
| **5** | 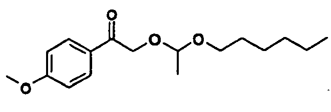 | acetanisole | hexyl acetate | ++ |
| **6** | 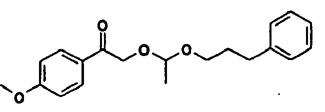 | acetanisole | phenylpropyl acetate | ++ |
| **7** | 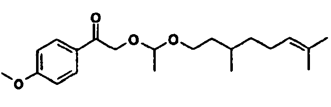 | acetanisole | citronellyl acetate | ++ |
| **8** | 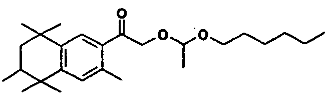 | Fixolide ^{®} | hexyl acetate | +++ |
| **9** | 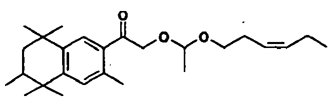 | Fixolide^{®} | cis-3-hexenyl acetate | +++ |
| **10** | 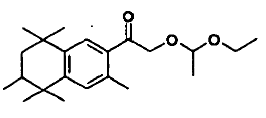 | Fixolide^{®} | (ethyl acetate) | +++ |
| **11** | 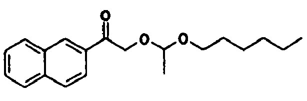 | Oranger Crystals^{®} | hexyl acetate | + |
| **12** | 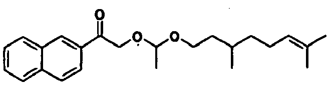 | Oranger Crystals^{®} | citronellyl acetate | + |
| **15** | 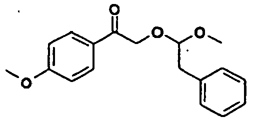 | acetanisole | methyl phenyl-acetate | ++ |

| | | | | |
|---|---|---|---|---|
| *0 : no cleavage, + : slow cleavage, ++ : medium cleavage, +++ : fast cleavage | | | | |

### Example 3

### Spray tests

1 g of an approximately 0.2% phenacyl acetal (I) solution in ethanol was evenly sprayed on a Terry towel (white cotton towel, 25cm x 25cm, 45 g), corresponding to 45-75 µg/g cotton. The sprayed towels were allowed to dry in a dark and odorless place. When dry, the towels were irradiated for a few seconds up to a few minutes with a tanning lamp (Osram Ultra-Vitalux^{®}, 300 W; at a distance of 50 cm, the light has approximately six to seven times the effect of the natural sunlight at noon on a sea-side midsummer day). The evaluation was done by a trained panel of perfumers before and after irradiation. Before irradiation, the towels were judged to be odorless. The results after irradiation are summarized in table 2.

**Table 2 : Release of aryl ketones (II) and esters (III) from phenacyl acetals (I) on fabrics upon irradiation with a tanning lamp**

| | STRUCTURE (I) | Fragrance Target (perception)* | | Global appreciation* |
|---|---|---|---|---|
| | | aryl ketone (II) | ester (III) | |
| **1** | 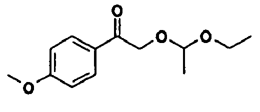 | acetanisole (++) | ethyl acetate (0) | + |
| **2** | 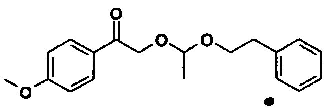 | acetanisole (++) | phenethyl acetate (+++) | +++ |
| **3** | 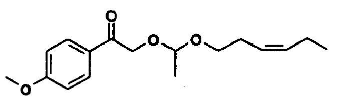 | acetanisole (++) | cis-3-hexenyl acetate (++) | ++ |
| **4** | 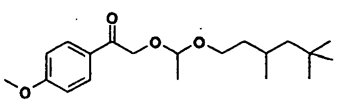 | acetanisole (++) | nonanyl acetate (+) | + |
| **5** | 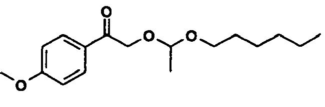 | acetanisole (++) | hexyl acetate (++) | ++ |
| **6** | 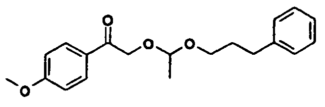 | acetanisole (++) | phenylpropyl acetate (++) | ++ |
| **7** | 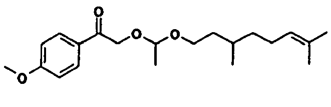 | acetanisole (++) | citronellyl acetate (+) | ++ |
| **8** | 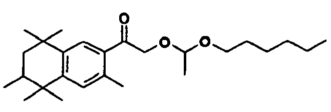 | Fixolide ^{®} (++) | hexyl acetate (+++) | +++ |
| **9** | 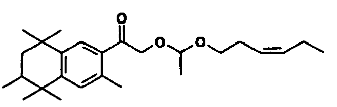 | Fixolide^{®} (++) | cis-3-hexenyl acetate (+++) | +++ |
| **10** | 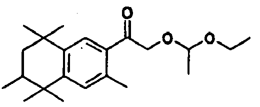 | Fixolide^{®} (++) | ethyl acetate (0) | ++ |
| **11** | 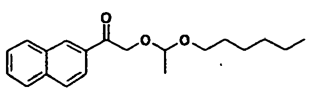 | Oranger Crystals^{®} (++) | hexyl acetate (++) | ++ |
| **12** | 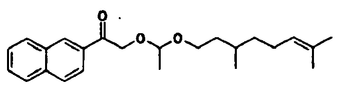 | Oranger Crystals^{®} (++) | citronellyl acetate (+) | ++ |
| **13** | 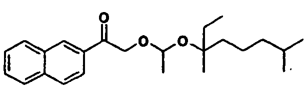 | Oranger Crystals ^{®} (++) | tetrahydro linalyl acetate (++) | +++ |
| **14** | 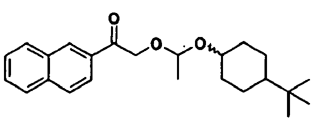 | Oranger Crystals^{®} (++) | *t*-butyl-cyclohexyl acetate (+) | + |
| **15** | 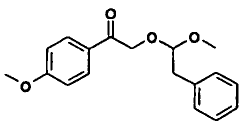 | acetanisole (++) | methyl phenyl acetate (+) | + |

| | | | | |
|---|---|---|---|---|
| * 0 : very weak, + : weak, ++ : medium, +++ : strong | | | | |

### Example 4

### Stability tests

The phenacyl acetals (I) were incubated in aqueous buffer solutions of pH 2.5, pH 7 and pH 9.5 for 24h at 37°C and were found to be stable in basic and neutral media, but less so under acidic conditions. The results are summarized in table 3.

**Table 3 : Stability of phenacyl acetals (III) under different pH**

| | STRUCTURE (I) | pH 2.5 | pH 7 | pH 9.5 | pH 11* |
|---|---|---|---|---|---|
| **1** | 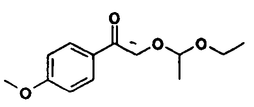 | unstable | stable | stable | not tested |
| **2** | 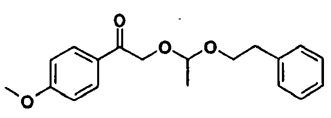 | unstable | stable | stable | stable |
| **3** | 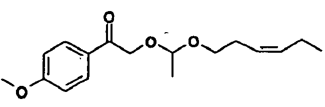 | unstable | stable | stable | stable |
| **4** | 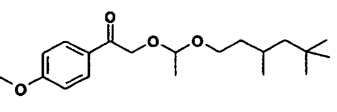 | unstable | stable | stable | stable |
| **5** | 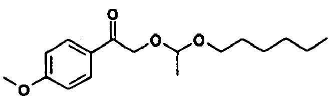 | unstable | stable | stable | stable |
| **6** | 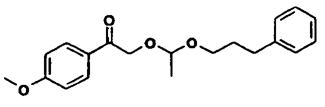 | unstable | stable | stable | stable |
| **8** | 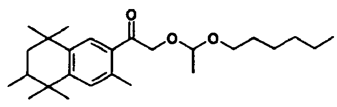 | stable | stable | stable | stable |
| **9** | 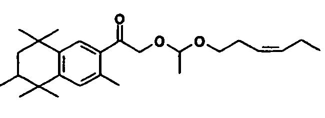 | stable | stable | stable | stable |

| | | | | | |
|---|---|---|---|---|---|
| * The results at pH 11 were determined under washing conditions as described in the example 5 below. | | | | | |

### Example 5

### Hand washing tests

Washing tests were performed according to the following hand washing test procedure with OMO Progress® base which contains the following ingredients:

| | |
|---|---|
| LAS | 22.0 % |
| STP | 13.3 % |
| CP5 (as 100%) | 1.5 % |
| SCMC (as 100%) | 0.34 % |
| Fluorescer (E1/1) | 1.41 |
| Savinase | 1.15 % |
| Lipolase | 0.15 % |
| Amilase | 0.30 % |
| Perborate | 8.0 % |
| TAED | 2.4 % |
| Alkalinity (pH) | 14.4 |

1- The washing powder (2.1 g) comprising the phenacyl acetal (I, about 21 mg, 1%) was dissolved in water (500 ml) at room temperature.
2- The towels (35 g) were added to the liquor and mixed with a glass stick.
3- Towels were soaked for 45 min with stirring every 15 min.
4- The wringed towels were rinsed three times with fresh water (250 ml) with intermediate wringing.
5- Towels were allowed to dry in a dark and odorless place before analysis or evaluation.

### Analysis

The towels were extracted with an organic solvent (preferably *t*-butyl methyl ether) using a Dionex ASE200 Accelerated Solvent Extractor and the extracts were analyzed by HPLC (Hewlett Packard Series 1100, column: Zorbax Eclipse XDB-C18, dimensions 15 cm x 4.6 mm x 5 µm). The washing liquor was extracted with an organic solvent (preferably t-butyl methyl ether, 250 ml) and analyzed by HPLC as above.

### Stability in washing liquor :

The washing liquor (2.1 g washing powder containing 1% phenacyl acetal (I) in 500 ml water), according to 1 in the above described washing procedure, was stirred during one hour at room temperature. Extraction with an organic solvent (preferably *t*-butyl methyl ether) to recover organic compounds and analysis with HPLC gave the amounts of recovered phenacyl acetal (I), table 4.

**Table 4: Stability of phenacyl acetals (I) under washing conditions**

| | STRUCTURE | Amount incorporated (mg) | Amount recovered (mg) | Recovered (mg) |
|---|---|---|---|---|
| **2** | 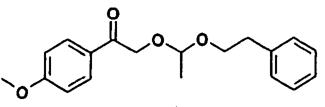 | 22.3 | 18.5 | 83 (±10) |
| **3** | 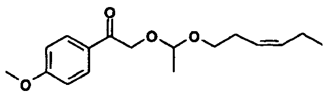 | 21 | 22.3 | 100 (±10) |
| **4** | 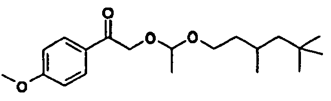 | 21.9 | 23.5 | 100 (±10) |
| **5** | 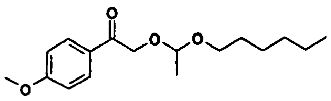 | 24.4 | 25.7 | 100 (±10) |
| **6** | 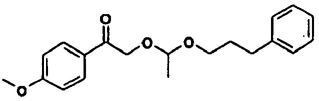 | 22.2 | 20.8 | 94 (±10) |
| **8** | 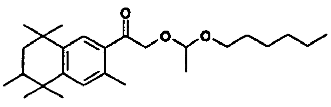 | 22.5 | 22.2 | 99 (±10) |
| **9** | 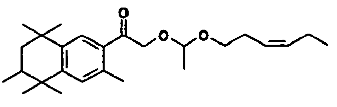 | 20.8 | 20.0 | 96 (±10) |

### Washing :

The dried towels, taken from the described hand washing procedure, were either irradiated with the previously mentioned tanning lamp and olfactively evaluated or analyzed by HPLC.
The aqueous liquors were extracted with an organic solvent (preferably *t*-butyl methyl ether) and analytical HPLC gave the results in table 5, which are related to partition between water and fabric.

**Table 5 : Partition between water and fabric (substantivity) of phenacyl acetals (I)**

| | STRUCTURE (I) | Amount incorporated (mg) | Amounts recovered | | | |
|---|---|---|---|---|---|---|
| | | | Water | | Towel extract | |
| | | | mg | % (±10%) | mg | % (±10%) |
| **2** | 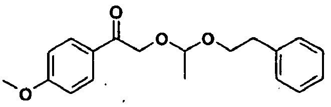 | 23.9 | 15 | 63 % | 10.5 | 44 % |
| **3** | 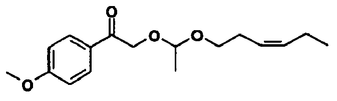 | 22.6 | 13.3 | 59 % | 14 | 62 % |
| **4** | 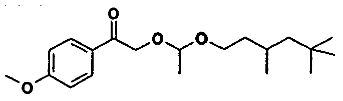 | 23.2 | 17.5 | 75 % | 8.9 | 38 % |
| **5** | 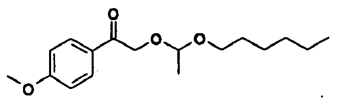 | 25.2 | 18.4 | 73 % | 11.7 | 46 % |
| **6** | 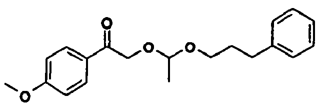 | 22.2 | 14.5 | 65 % | 17 | 47 % |
| **8** | 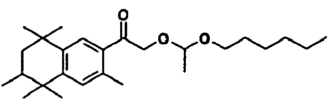 | 20.6 | 16.3 | 69 % | 8.7 | 42 % |
| **9** | 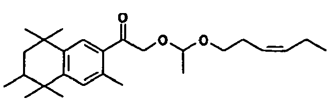 | 22.8 | 15.3 | 67 % | 10.3 | 45 % |

## Claims

1. Use as a fragrance precursor of a compound of formula I 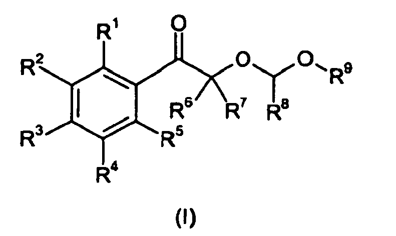 for a fragrant ketone of formula II 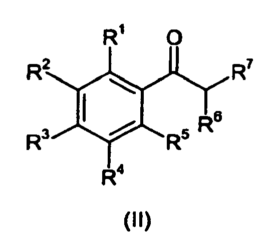 and a fragrant ester of formula III 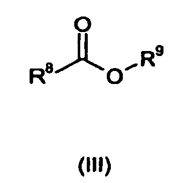
R¹ to R⁵ represent independently H, -NO₂, linear or branched C₁-C₆-alkyl, C₁-C₆-alkenyl, C₁-C₆-alkinyl or C₁-C₄-alkoxy,
R¹ and R², R² and R³, R³ and R⁴ and R⁴ and R⁵ may form together one or two aliphatic or aromatic rings, these rings may optionally contain linear or branched C₁-C₄-alkyl, C₁-C₄-alkenyl or C₁-C₄-alkiriyl residues, and the above rings and residues may comprise one or more oxygen atoms,
R⁶ and R⁷ are independently H, linear or branched C₁-C₆-alkyl-, C₁-C₆-alkenyl, C₁-C₆-alkinyl, and R⁶ or R⁷ may form with either R¹ or R⁵ a carbocyclic ring optionally substituted by an aliphatic residue
R⁸ and R⁹ are respectively the residues of an aliphatic acid R⁸-COOH having up to 4 carbon atoms, but not only one carbon atom and an aliphatic alcohol R⁹OH having 2 to 20 carbon atoms, forming the fragrant ester of formula III.

2. Use according to claim 1, wherein R⁸ is -CH₃ and R⁹ is the residue of an aliphatic alcohol having 2 to 20 carbon atoms or an araliphatic alcohol having more than 5 carbon atoms.

3. Use according to claim 1, wherein R⁸ is the residue of an aliphatic acid having 5 to 20 carbon atoms and R⁹ is the residue of an aliphatic alcohol having 1 to 5 carbon atoms, R⁹ being preferably -CH₂CH₃.

4. Use according to claim 1, wherein R⁸ is the residue of an aliphatic acid having up to 4 carbon atoms, but not only one carbon atom and R⁹ is the residue selected from a residue of (a) a terpene alcohol having 10 to 20 carbon atoms; (b) a monoterpene alcohol; and (c) an araliphatic alcohol having more than 5 carbon atoms.

5. Use according to claim 1, wherein R⁸ is the residue of a cycloaliphatic acid having 5 to 20 carbon atoms and R⁹ is the residue of aliphatic alcohol having 1 to 5 carbon atoms, R⁹ preferably being -CH₂CH₃.

6. Use according to claim 1, wherein at least one of the residues R⁶ and R⁷, is H, preferably both residues R⁶ and R⁷ being H.

7. Use according to claim 1, wherein R⁶ and R⁷ are H and R¹ to R⁵ represent independently H, -NO₂, linear or branched C₁-C₆-alkyl, C₁-C₆-alkenyl, C₁-C₆-alkinyl or C₁-C₄ alkoxy.

8. Use according to claim 1, wherein R¹ and R², R² and R³, R³ and R⁴, R⁴ and R⁵, form together an aliphatic or aromatic ring which may optionally contain substituted or unsubstituted C₁-C₄-alkyl, C₁-C₄-alkenyl, C₁-C₄-alkinyl residues and may comprise one or more oxygen atoms.

9. A compound of formula I 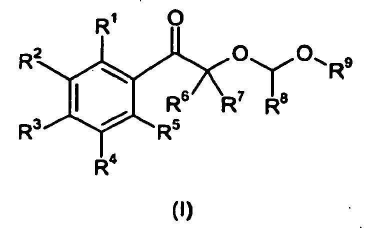 wherein
R¹ to R⁵ represent independently H, -NO₂, linear or branched C₁-C₆-alkyl, C₁-C₆-alkenyl, C₁-C₆-alkinyl, or C₁-C₄-alkoxy,
R¹ and R², R² and R³, R³ and R⁴ and R⁴ and R⁵ may form together one or two aliphatic or aromatic rings, these rings may optionally contain substituted or unsubstituted C₁-C₄-alkyl, C₁-C₄-alkenyl or C₁-C₄-alkinyl residues, and may comprise one or more oxygen atoms,
R⁶ and R⁷ are independently H, linear or branched C₁-C₆-alkyl, C₁-C₆-alkenyl, C₁-C₆-alkinyl, and R⁶ or R⁷ may form with either R¹ or R⁵ a substituted or unsubstituted carbocyclic ring, and R⁸ and R⁹ are respectively the residues of an aliphatic acid R⁸-COOH having up to 4 carbon atoms, but not only one carbon atom and an aliphatic alcohol R⁹OH having 2 to 20 carbon atoms, which together form a fragrant ester.

10. A product comprising a fragrance precursor, which is a compound of the type described in claim 1.

## Patentansprüche

1. Verwendung von einer Verbindung der Formel 1 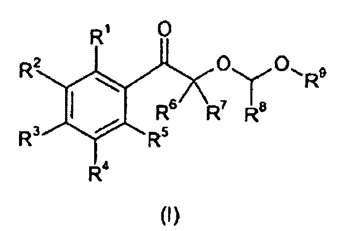 als Duftvorläufer für ein wohl riechendes Keton der Formel II 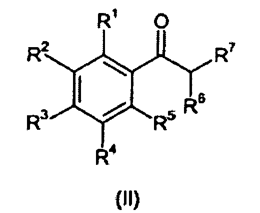 und einen wohl riechenden Ester der Formel III 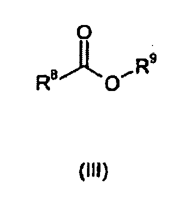 wobei
R¹ bis R⁵ unabhängig stehen für H, -NO₂, lineares oder verzweigtes C₁-C₆-Alkyl, C₁-C₆-Alkenyl, C₁-C₆-Alkinyl oder C₁-C₄-Alkoxy,
R¹ und R², R² und R³, R³ und R⁴ und R⁴ und R⁵ zusammen ein oder zwei aliphatische oder aromatische Ringe bilden können, wobei diese Ringe gegebenenfalls lineare oder verzweigte C₁-C₄-Alkyl-, C₁-C₄-Alkenyl-, C₁-C₄-Alkinylreste enthalten können, und die obigen Ringe und Reste ein oder mehr Sauerstoffatome umfassen können,
R⁶ und R⁷ unabhängig stehen für H, lineares oder verzweigtes C₁-C₆-Alkyl, C₁-C₆-Alkenyl, C₁-C₆-Alkinyl, und R⁶ oder R⁷ mit entweder R¹ oder R⁵ einen carbocyclischen Ring bilden können, der gegebenenfalls substituiert ist durch einen aliphatischen Rest,
R⁸ und R⁹ jeweils für die Reste stehen von einer aliphatischen Säure R⁸-COOH, die bis zu vier Kohlenstoffatome aufweist, aber nicht nur ein Kohlenstoffatom, und von einem aliphatischen Alkohol R⁹OH, der 2 bis 20 Kohlenstoffatome aufweist, die den wohl riechenden Ester der Formel III bilden.

2. Verwendung nach Anspruch 1, wobei R⁸ für -CH₃ steht, und R⁹ für den Rest eines aliphatischen Alkohols steht, der 2 bis 20 Kohlenstoffatome aufweist, oder eines araliphatischen Alkohols, der mehr als fünf Kohlenstoffatome aufweist.

3. Verwendung nach Anspruch 1, wobei R⁸ für den Rest von einer aliphatischen Säure steht, die 5 bis 20 Kohlenstoffatome aufweist, und R⁹ für den Rest von einem aliphatischen Alkohol steht, der 1 bis 5 Kohlenstoffatome aufweist, wobei R⁹ vorzugsweise für -CH₂CH₃ steht.

4. Verwendung nach Anspruch 1, wobei R⁸ für den Rest von einer aliphatischen Säure steht, die bis zu vier Kohlenstoffatome aufweist, aber nicht nur ein Kohlenstoffatom, und R⁹ für den Rest steht, der ausgewählt ist aus einem Rest von (a) einem Terpenalkohol, der 10 bis 20 Kohlenstoffatome aufweist; (b) einem Monoterpenalkohol; und (c) einem araliphatischen Alkohol, der mehr als fünf Kohlenstoffatome aufweist.

5. Verwendung nach Anspruch 1, wobei R⁸ für den Rest einer cycloaliphatischen Säure steht, die 5 bis 20 Kohlenstoffatome aufweist, und R⁹ für den Rest eines aliphatischen Alkohols steht, der 1 bis 5 Kohlenstoffatome aufweist, wobei R⁹ vorzugsweise für -CH₂CH₃ steht.

6. Verwendung nach Anspruch 1, wobei mindestens einer der Reste R⁶ und R⁷ für H steht, vorzugsweise beide Reste R⁶ und R⁷ für H stehen.

7. Verwendung nach Anspruch 1, wobei R⁶ und R⁷ für H stehen, und R¹ bis R⁵ unabhängig stehen für H, -NO₂ lineares oder verzweigtes C₁-C₆-Alkyl, C₁-C₆-Alkenyl, C₁-C₆-Alkinyl oder C₁-C₄-Alkoxy.

8. Verwendung nach Anspruch 1, wobei R¹ und R², R² und R³, R³ und R⁴, R⁴ und R⁵ zusammen einen aliphatischen oder aromatischen Ring bilden, der gegebenenfalls substituierte oder unsubstituierte C₁-C₄-Alkyl-, C₁-C₄-Alkenyl-, C₁-C₄-Alkinylreste und ein oder mehr Sauerstoffatome umfassen kann.

9. Verbindung der Formel I 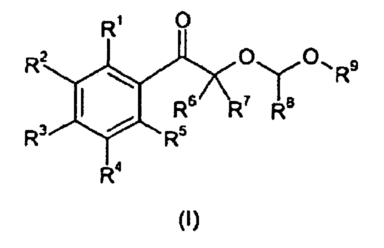 wobei
R¹ bis R⁵ unabhängig stehen für H, -NO₂, lineares oder verzweigtes C₁-C₆-Alkyl, C₁-C₆-Alkenyl, C₁-C₆-Alkinyl oder C₁-C₄-Alkoxy,
R¹ und R², R² und R³, R³ und R⁴ und R⁴ und R⁵ zusammen ein oder zwei aliphatische oder aromatische Ringe bilden können, wobei diese Ringe gegebenenfalls lineare oder verzweigte C₁-C₄-Alkyl-, C₁-C₄-Alkenyl- oder C₁-C₄-Alkinylreste enthalten können und ein oder mehr Sauerstoffatome umfassen können,
R⁶ und R⁷ unabhängig stehen für H, lineares oder verzweigtes C₁-C₆-Alkyl, C₁-C₆-Alkenyl, C₁-C₆-Alkinyl, und R⁶ oder R⁷ mit entweder R¹ oder R⁵ einen substituierten oder unsubstituierten carbocyclischen Ring bilden kann, und R⁸ und R⁹ jeweils für die Reste stehen von einer aliphatischen Säure R⁸-COOH, die bis zu vier Kohlenstoffatome aufweist, aber nicht nur ein Kohlenstoffatom, und von einem aliphatischen Alkohol R⁹OH, der 2 bis 20 Kohlenstoffatome aufweist, die zusammen einen wohl riechenden Ester bilden.

10. Erzeugnis, umfassend einen Duftvorläufer, der eine Verbindung von dem Typ ist, wie in Anspruch 1 beschrieben.

## Revendications

1. Utilisation, en tant que précurseur de parfum, d'un composé de formule I : 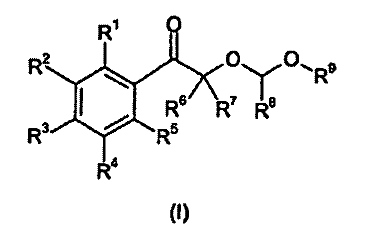 pour une cétone aromatique de formule II : 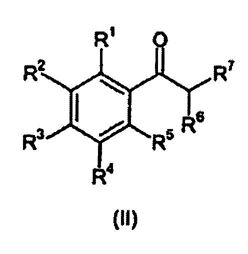 et un ester aromatique de formule III : 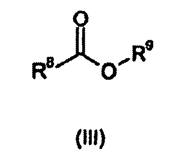
les résidus R¹ à R⁵ représentent indépendamment H, -NO₂, un groupe alkyle en C₁ à C₆ linéaire ou ramifié, alcényle en C₁ à C₆, alcynyle en C₁ à C₆ ou alcoxy en C₁ à C₄,
les résidus R¹ et R², R² et R³, R³ et R⁴, et R⁴ et R⁵ peuvent former conjointement un ou deux cycles aliphatiques ou aromatiques, ces cycles pouvant contenir facultativement des résidus alkyle en C₁ à C₄ linéaire ou ramifié, alcényle en C₁ à C₄ ou alcynyle en C₁ à C₄, et les cycles et résidus ci-dessus pouvant comprendre un ou plusieurs atomes d'oxygène, les résidus R⁶ et R⁷ représentent indépendamment H, un groupe alkyle en C₁ à C₆ linéaire ou ramifié, alcényle en C₁ à C₆, alcynyle en C₁ à C₆, et R⁶ ou R⁷ peut former, soit avec R¹, soit avec R⁵, un cycle carbocyclique facultativement substitué par un résidu aliphatique,
les résidus R⁸ et R⁹ représentent respectivement les résidus d'un acide aliphatique R⁸-COOH ayant jusqu'à 4 atomes de carbone, mais pas uniquement un atome de carbone, et d'un alcool aliphatique R⁹OH ayant 2 à 20 atomes de carbone, formant l'ester aromatique de formule III.

2. Utilisation selon la revendication 1, dans laquelle R⁸ représente -CH₃ et R⁹ est le résidu d'un alcool aliphatique ayant 2 à 20 atomes de carbone ou d'un alcool araliphatique ayant plus de 5 atomes de carbone.

3. Utilisation selon la revendication 1, dans laquelle R⁸ est le résidu d'un acide aliphatique ayant 5 à 20 atomes de carbone, et R⁹ est le résidu d'un alcool aliphatique ayant 1 à 5 atomes de carbone, R⁹ représentant de préférence -CH₂CH₃.

4. Utilisation selon la revendication 1, dans laquelle R⁸ est le résidu d'un acide aliphatique ayant jusqu'à 4 atomes de carbone, mais pas seulement un seul atome de carbone, et R⁹ est le résidu choisi entre un résidu de (a) un alcool terpénique ayant 10 à 20 atomes de carbone ; (b) un alcool monoterpénique ; et (c) un alcool araliphatique ayant plus de 5 atomes de carbone.

5. Utilisation selon la revendication 1, dans laquelle R⁸ est le résidu d'un acide cycloaliphatique ayant 5 à 20 atomes de carbone et R⁹ est le résidu d'un alcool aliphatique ayant 1 à 5 atomes de carbone, R⁹ représentant de préférence -CH₂CH₃.

6. Utilisation selon la revendication 1, dans laquelle au moins l'un des résidus R⁶ et R⁷ représente H, de préférence les deux résidus R⁶ et R⁷ représentant H.

7. Utilisation selon la revendication 1, dans laquelle les résidus R⁶ et R⁷ représentent H et les résidus R¹ à R⁵ représentent indépendamment H, -NO₂, un groupe alkyle en C₁ à C₆ linéaire ou ramifié, alcényle en C₁ à C₆, alcynyle en C₁ à C₆ ou alcoxy en C₁ à C₄.

8. Utilisation selon la revendication 1, dans laquelle les radicaux R¹ et R², R² et R³, R³ et R⁴, R⁴ et R⁵, forment conjointement un cycle aliphatique ou aromatique qui peut contenir facultativement des résidus substitués ou non substitués alkyle en C₁ à C₄, alcényle en C₁ à C₄, alcynyle en C₁ à C₄, et peut comprendre un ou plusieurs atomes d'oxygène.

9. Composé de formule I : 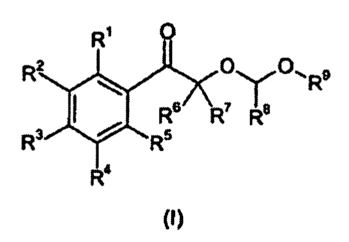
dans laquelle les résidus R¹ à R⁵ représentent indépendamment H, -NO₂, un groupe alkyle en C₁ à C₆ linéaire ou ramifié, alcényle en C₁ à C₆, alcynyle en C₁ à C₆ ou alcoxy en C₁ à C₄,
Les résidus R¹ et R², R² et R³, R³ et R⁴, et R⁴ et R⁵ peuvent former conjointement un ou deux cycles aliphatiques ou aromatiques, ces cycles pouvant contenir facultativement des résidus substitués ou non substitués alkyle en C₁ à C₄, alcényle en C₁ à C₄ ou alcynyle en C₁ à C₄, et pouvant comprendre un ou plusieurs atomes d'oxygène,
les résidus R⁶ et R⁷ représentent indépendamment H, un groupe alkyle en C₁ à C₆ linéaire ou ramifié, alcényle en C₁ à C₆, alcynyle en C₁ à C₆, et R⁶ ou R⁷ peut former soit avec R¹, soit avec R⁵, un cycle carbocyclique substitué ou non substitué, et R⁸ et R⁹ sont respectivement les résidus d'un acide aliphatique R⁸-COOH ayant jusqu'à 4 atomes de carbone, mais pas uniquement un atome de carbone, et un alcool aliphatique R⁹OH ayant 2 à 20 atomes de carbone, qui forment conjointement un ester aromatique.

10. Produit comprenant un précurseur de parfum, qui est un composé du type décrit dans la revendication 1.
